# EUROPEAN PATENT SPECIFICATION

(11) **EP 3 946 530 B1**
(45) Date of publication and mention of the grant of the patent: **05.11.2025**
(21) Application number: 20715116.8
(22) Date of filing: 20.03.2020
(51) Int. Cl.: A61M 16/00, A61B 5/00, A61M 16/20

(54) **RESPIRATORY THERAPY APPARATUS**
ATEMTHERAPIEVORRICHTUNG
APPAREIL D'INHALOTHÉRAPIE

(30) Priority: 05.04.2019 GB 201904825
(43) Date of publication of application: 09.02.2022
(73) Proprietor: ICU Medical International Limited, Lower Pemberton Ashford, Kent TN25 4BF (GB)
(72) Inventor: HANIF, Usumah, Kent TN24 0TZ (GB); KHASAWNEH, Mohammad, Qassim, Mohammad, Canterbury CT1 2FR (GB); BASU, Samit, K., Freemont CA 94538 (GB)
(74) Representative: Flint, Jonathan McNeill
(86) International application number: PCT/GB2020/000033
(87) International publication number: WO 2020/201665

(56) References cited:
- WO-A1-2013/043847
- WO-A1-2014/202923
- US-A1- 2018 008 790
- US-B1- 6 581 598
- US-B2- 7 402 139

## Description

This invention relates to respiratory therapy apparatus of the kind including an oscillatory respiratory therapy device with a displaceable member displaced by breathing through the device and arranged to produce an oscillating resistance to breathing through the device.

Positive expiratory pressure (PEP) apparatus, that is, apparatus that presents a resistance to expiration through the device, are now widely used to help treat patients suffering from a range of respiratory impairments, such as chronic obstructive pulmonary disease, bronchitis, cystic fibrosis and atelectasis. More recently, such apparatus that provide an alternating resistance to flow have been found to be particularly effective. One example of such apparatus is sold under the trade mark Acapella (a registered trade mark of Smiths Medical) by Smiths Medical and is described in US6581598, US6776159, US7059324 and US7699054. Other vibratory respiratory therapy apparatus is available, such as "Quake" manufactured by Thayer, "AeroPEP" manufactured by Monaghan, "TheraPEP" manufactured by Smiths Medical and "IPV Percussionator" manufactured by Percussionaire Corp. Alternative apparatus such as "CoughAssist" manufactured by Philips is also available. Respiratory therapy apparatus can instead provide an alternating resistance to flow during inhalation.

To be effective these apparatus must be used regularly at prescribed intervals. In the case of chronic diseases, the patient needs to use the apparatus daily for the rest of his life in order to maintain a continuous relief.

Although these apparatus can be very effective, users often neglect to use the apparatus regularly at the prescribed frequency. It is very difficult to maintain a record of use of the apparatus, especially when the patient is using it at home. The clinician often does not know whether deterioration in a patient's condition is because he has failed to use the apparatus as prescribed or whether other factors are the cause. WO2014/202923 describes apparatus including an acoustic sensor such as a microphone responsive to the sound produced by the therapy device. Other apparatus is described, for example, in WO2014/202924, WO2015/036723, WO2015/104522, WO2015/114285, WO2015/198001, WO2016/075426, WO2016/092247, WO2017/178776, WO2017/194906, WO2017/187116, GB2560105 and WO2019/243758. US7402139 describes apparatus for determining the impedance of a patient's respiratory tract by using an electro-acoustic device to generate an oscillating signal. WO2013/043847 describes an arrangement employing diaphragm sensors to detect breath through mouthpiece.

It is an object of the present invention to provide alternative respiratory therapy apparatus. The invention is defined by the appended claims.

According to the present invention there is provided a respiratory therapy apparatus of the above-specified kind, characterised in that the apparatus includes an electronic camera responsive to displacement of the displaceable member, that a part at least of the displaceable member is marked with a contrasting colour or a reflective surface to improve sensitivity of the camera to displacement of the displaceable member, that the camera is provided externally of the device, and that the device has a transparent housing or a transparent window through which the camera can view the displaceable member in the device.

The camera is preferably arranged to provide a signal indicative of one or more of the following: when the device is used, the duration of use, and the frequency of oscillation. The camera may contained in a unit including a display on which is represented the frequency of oscillation detected by the sensor. The camera may be provided in a phone, the phone being programmed to respond to displacement of the displaceable member in the device during use. The displaceable member may include a valve element on a rocker arm that opens and closes an opening during exhalation through the device. The apparatus may also include a microphone arranged to sense the sound made by the therapy device, the apparatus combining signals from the camera and microphone to improve the signal to noise ratio. The camera may be arranged to provide an indication of the range of the therapy device from the camera, the gain of the apparatus being adjusted accordingly.

Apparatus including an oscillatory PEP device will now be described, by way of example, with reference to the accompanying drawings, in which:
- Figure 1: is an exploded view of the apparatus;
- Figure 2: illustrates the apparatus in use; and
- Figure 3: is a front elevation view of one form of a dedicated sensor.

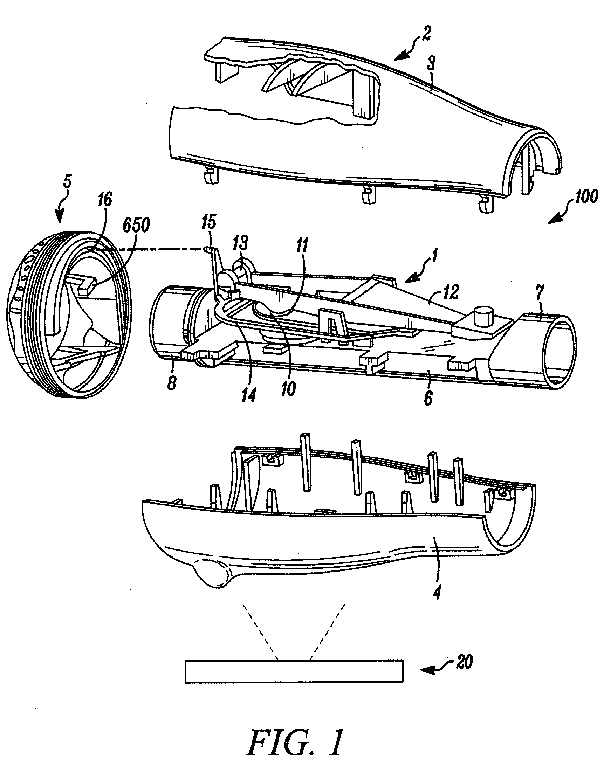

With reference first to Figure 1, the device 100 comprises a rocker assembly 1 contained within an outer, optically-transparent housing 2 provided by an upper part 3 and a lower part 4 of substantially semi-cylindrical shape. The device is completed by an adjustable dial 5 of circular section. The rocker assembly 1 includes an air flow tube 6 with a breathing inlet 7 at one end and an inspiratory inlet 8 at the opposite end including a one-way valve (not shown) that allows air to flow into the air flow tube 6 but prevents air flowing out through the inspiratory inlet. The air flow tube 6 has an outlet opening 10 with a non-linear profile that is opened and closed by a conical valve element 11 mounted on a rocker arm 12 pivoted midway along its length about a transverse axis. The air flow tube 6 and housing 2 provide a structure with which the rocker arm 12 is mounted. At its far end, remote from the breathing inlet 7, the rocker arm 12 carries an iron pin 13 that interacts with the magnetic field produced by a permanent magnet (not visible) mounted on an adjustable support frame 14. The magnet arrangement is such that, when the patient is not breathing through the device, the far end of the rocker arm 12 is held down such that its valve element 11 is also held down in sealing engagement with the outlet opening 10. A cam follower projection 15 at one end of the support frame 14 locates in a cam slot 16 in the dial 5 such that, by rotating the dial, the support frame 14, with its magnet, can be moved up or down to alter the strength of the magnetic field interacting with the iron pin 13. The dial 5 enables the frequency of operation and the resistance to flow of air through the device to be adjusted for maximum therapeutic benefit to the user.

When the patient inhales through the breathing inlet 7 air is drawn through the inspiratory inlet 8 and along the air flow tube 6 to the breathing inlet. When the patient exhales, the one-way valve in the inspiratory inlet 8 closes, preventing any air flowing out along this path. Instead, the expiratory pressure is applied to the underside of the valve element 11 on the rocker arm 12 causing it to be lifted up out of the opening 10 against the magnetic attraction, thereby allowing air to flow out to atmosphere. The opening 10 has a non-linear profile, which causes the effective discharge area to increase as the far end of the rocker arm 12 lifts, thereby allowing the arm to fall back down and close the opening. As long as the user keeps applying sufficient expiratory pressure, the rocker arm 12 will rise and fall repeatedly as the opening 10 is opened and closed, causing a vibratory, alternating or oscillating resistance to expiratory breath flow through the device. Further information about the construction and operation of the device can be found in US6581598, but is not essential for an understanding of the present invention.

As so far described, the apparatus is conventional.

The apparatus of the present invention includes the device 100 described above and a camera 20 responsive to movement of the rocker arm 12 caused breathing through the device.

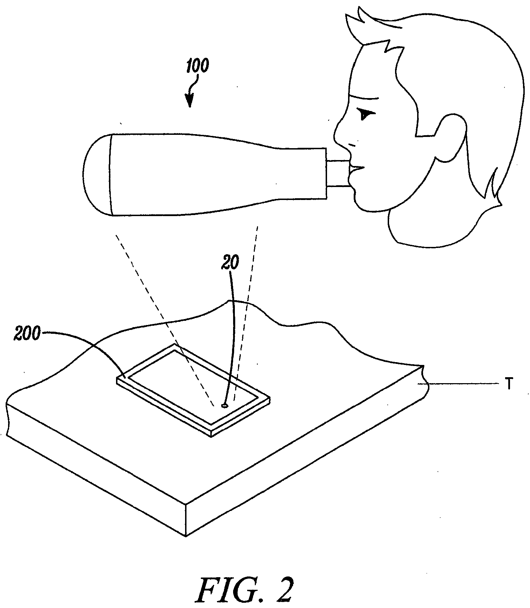

Figure 2 shows a device 100 and an electronic camera 20 in a conventional mobile phone 200. As shown, the phone 200 might conveniently be placed on the top of a table T at which the user is sitting so that it lies beneath the therapy device 100 when this is held up to the mouth during use.

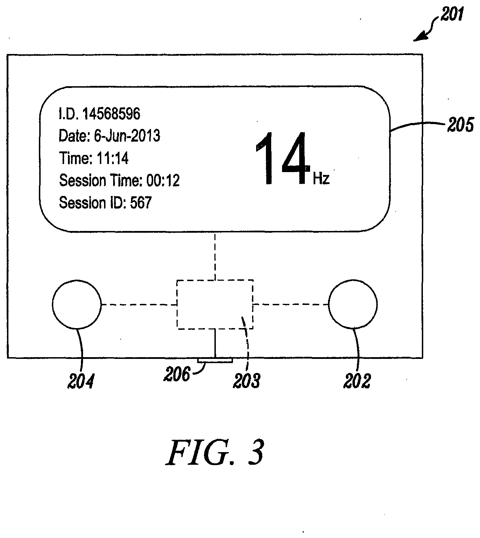

Figure 3 shows an alternative optical sensor provided by a stand-alone camera unit 201 that is separate from the device 100 but, in use, is placed close to it. The camera unit 201 includes a conventional electronic optical video sensor 202 connected to a processing and memory unit 203, which is also connected to an on/off button 204 and a display screen 205. The unit 201 also has a data interface, such as the USB port 206 shown, or a wireless interface, such as a radio frequency Bluetooth or an infra-red interface. The unit 201 may also include input means by which information can be entered to the sensor unit, or this could be carried out via the data interface 206 from a remote computer or the like.

In use, the mobile phone 200 or stand-alone camera unit 201 is placed close to the device 100, so that displacement of the rocker arm 12 is within the visible range of the camera, but is not in direct contact with the device. Typically, the phone 200 or other unit 201 would be placed within about 25cm of the therapy device 100. The stand-alone unit 201 is turned on using the button 204, thereby causing the display 205 to show a representation of the user's identification, such as in the form of a unique number, the date and present time. When the user starts the therapy session the rocker arm 12 moves up and down as the user exhales through the device 100. The output of the camera unit 201 is appropriately processed by the processing unit 203 to extract a signal indicative of the frequency of oscillation of the rocker arm 12. The processing unit 203 can measure various parameters, such as the duration of each exhalation, the number of exhalations in each session, and the oscillation frequency during exhalation. As illustrated in Figure 3, the screen 205 provides the user with a representation of the session time and the frequency of oscillation, which has been found to be particularly important to users in achieving the best therapeutic effect. The sensor unit 201 or phone 200 preferably also provides the user with feedback as to whether he has achieved his target oscillation frequency. This may be done in various different ways, such as by displaying a legend on the display: "Flow OK", "Flow Too High" or "Flow Too Low". Alternatively, the screen 205, or a part of the screen, could change colour to indicate whether flow was too high or low, or a sound signal could be produced.

As mentioned above, the setting of the dial 5 on the therapy device 100 affects the frequency and resistance to flow through the device. This is set by the user to achieve the maximum beneficial effect. The phone 200 or camera unit 201 could be arranged to compute a measure of the flow rate and pressure generated from the measured frequency and from knowledge of the setting of the dial 5, as entered into the phone or unit by the user or clinician.

The camera sensor unit 200, 201 can be tuned to be sensitive to oscillations in a relatively small range of frequencies. By filtering out other frequencies it is possible to use high gain amplification for maximum sensitivity.

The phone 200 and sensor unit 201 described above are both separate from the therapy device 100. However, a sensor unit could be mounted with the therapy device, such as by means of a clip or strap that supports the sensor on the device.

The camera need not be responsive to radiation in the visible part of the spectrum but could be responsive to other optical radiation, such as infra-red or ultra-violet radiation. Where the camera needs to respond to movement of the displaceable member within the housing of the therapy device it will be appreciated that the housing, or a window, would need to be transparent to the radiation being sensed, that is, to visible, infra-red or ultra-violet radiation.

Instead of being incorporated into a mobile phone, the camera unit could be provided as a program application in a general purpose computer, using the camera built into the computer, or a separate plug-in camera. The program application could be arranged to stop automatically after the elapse of a predetermined time without sensing any movement having the appropriate characteristics.

In the arrangement described above the camera is positioned to sense the up and down rocking movement of the rocker arm 12 through the transparent housing 2. It is not essential that the housing be entirely transparent if there is a transparent window through which the rocker arm or other displaceable member is visible. In order to improve sensitivity of the camera it might be desirable to mark a part or all of the rocker arm with a contrasting colour or a reflective surface. Alternatively, the displaceable member in the therapy device could be mechanically linked to a flag on the outside the casing of the device so that the flag moves with the displaceable member and can be viewed by the camera.

It will be appreciated that there are many different ways in which information obtained by the camera unit can be represented so that it is provided to the user and clinician in the most useful manner.

Apparatus of the present invention can be used with any conventional oscillatory respiratory therapy device having a displaceable member the movement of which can be viewed optically either directly or indirectly. The therapy device may be combined with other treatments such as nebulisation or the administration of aerosol medication.

The camera could be combined with an arrangement including a microphone that senses the sound made by the therapy device, such as of the kind described in WO2014/202923. By combining the optical and acoustic signals the signal to noise ratio of the apparatus can be improved. The camera could be used to provide an indication of the range of the therapy device from the sensor, the apparatus being arranged to adjust its gain appropriately.

Because the camera is separate from the therapy device it enables the device to be cleaned periodically without the risk of damaging any electrical components in the camera. It also enables the device to be disposed of at the end of its life without the need to dispose of the camera, which can be reused with a replacement therapy device.

The present invention enables apparatus to be provided that gives useful data about use of an existing, conventional therapy device with little or no modification of the device itself. In this way, the user can be made more aware of how well he is complying with the prescribed therapy programme and can modify his use of the device accordingly to achieve maximum benefit. The clinician is also able to check patient compliance so that he can identify whether any deterioration in a patient's condition is due to lack of compliance or if alternative treatment is needed. The therapy device need not be an expiratory therapy device but could, for example be an inspiratory therapy device.

## Claims

1. Respiratory therapy apparatus including an oscillatory respiratory therapy device (100) with a displaceable member (11, 12) displaced by breathing through the device and arranged to produce an oscillating resistance to breathing through the device, **characterised in that** the apparatus includes an electronic camera (20, 202) responsive to displacement of the displaceable member (10, 12), that a part at least of the displaceable member (11, 12) is marked with a contrasting colour or a reflective surface to improve sensitivity of the camera (20, 202) to displacement of the displaceable member, that the camera (20, 202) is provided externally of the device (100), and that the device has a transparent housing (2) or a transparent window through which the camera (20, 202) can view the displaceable member (11, 12) in the device.

2. Respiratory therapy apparatus according to Claim 1, **characterised in that** the camera (20, 202) is arranged to provide a signal indicative of one or more of the following: when the device (100) is used, the duration of use, and the frequency of oscillation.

3. Respiratory therapy apparatus according to any one of the preceding claims, **characterised in that** the camera (20, 202) is contained in a unit (200, 201) including a display (205) on which is represented the frequency of oscillation detected by the camera.

4. Respiratory therapy apparatus according to Claim 3, **characterised in that** the camera is provided in a phone (200), and that the phone is programmed to respond to displacement of the displaceable member (11, 12) in the device (100) during use.

5. Respiratory therapy apparatus according to any one of the preceding claims, **characterised in that** the displaceable member includes a valve element (11) on a rocker arm (12) that opens and closes an opening during exhalation through the device (100).

6. Respiratory therapy apparatus according to any one of the preceding claims, **characterised in that** the apparatus also includes a microphone arranged to sense the sound made by the therapy device, and that the apparatus combines signals from the camera (20, 202) and microphone to improve the signal to noise ratio.

7. Respiratory therapy apparatus according to any one of the preceding claims, **characterised in that** the camera (20, 202) is arranged to provide an indication of the range of the therapy device (100) from the camera, and that the apparatus is arranged to adjust its gain accordingly.

## Patentansprüche

1. Atemtherapiegerät mit einer oszillierenden Atemtherapievorrichtung (100) mit einem durch Atmung durch die Vorrichtung verschiebbaren Element (11, 12), das so angeordnet ist, dass es einen oszillierenden Widerstand gegen die Atmung durch die Vorrichtung erzeugt, **dadurch gekennzeichnet, dass** das Gerät eine elektronische Kamera (20, 202) umfasst, die auf die Verschiebung des verschiebbaren Elements reagiert (10, 12), dass zumindest ein Teil des verschiebbaren Elements (11, 12) mit einer Kontrastfarbe oder einer reflektierenden Oberfläche markiert ist, um die Empfindlichkeit der Kamera (20, 202) gegenüber einer Verschiebung des verschiebbaren Elements zu verbessern, dass die Kamera (20, 202) außerhalb der Vorrichtung (100) vorgesehen ist, und dass die Vorrichtung ein transparentes Gehäuse (2) oder ein transparentes Fenster aufweist, durch das die Kamera (20, 202) das verschiebbare Element (11, 12) in der Vorrichtung sehen kann.

2. Atemtherapiegerät nach Anspruch 1, **dadurch gekennzeichnet, dass** die Kamera (20, 202) so angeordnet ist, dass sie ein Signal liefert, das eines oder mehrere der folgenden Merkmale anzeigt: wenn das Gerät (100) verwendet wird, die Dauer der Verwendung und die Schwingungsfrequenz.

3. Atemtherapiegerät nach einem der vorstehenden Ansprüche, **dadurch gekennzeichnet, dass** die Kamera (20, 202) in einer Einheit (200, 201) enthalten ist, die eine Anzeige (205) umfasst, auf der die von der Kamera erfasste Schwingungsfrequenz dargestellt wird.

4. Atemtherapiegerät nach Anspruch 3, **dadurch gekennzeichnet, dass** die Kamera in einem Telefon (200) vorgesehen ist und dass das Telefon so programmiert ist, dass es auf eine Verschiebung des verschiebbaren Elements (11, 12) in der Vorrichtung (100) während des Gebrauchs reagiert.

5. Atemtherapiegerät nach einem der vorstehenden Ansprüche, **dadurch gekennzeichnet, dass** das verschiebbare Element ein Ventilelement (11) an einem Kipphebel (12) umfasst, dass während der Ausatmung durch die Vorrichtung (100) eine Öffnung öffnet und schließt.

6. Atemtherapiegerät nach einem der vorstehenden Ansprüche, **dadurch gekennzeichnet, dass** das Gerät außerdem ein Mikrofon umfasst, das so angeordnet ist, dass es den von der Therapievorrichtung erzeugten Schall erfasst, und dass das Gerät Signale von der Kamera (20, 202) und dem Mikrofon kombiniert, um das Signal-Rausch-Verhältnis zu verbessern.

7. Atemtherapiegerät nach einem der vorstehenden Ansprüche, **dadurch gekennzeichnet, dass** die Kamera (20, 202) so angeordnet ist, dass sie eine Hinweis des Abstands des Therapiegeräts (100) von der Kamera liefert, und dass das Gerät so angeordnet ist, dass es seine Verstärkung entsprechend anpasst.

## Revendications

1. Appareil de thérapie respiratoire comprenant un dispositif de thérapie respiratoire oscillatoire (100) avec un élément déplaçable (11, 12) déplacé par la respiration à travers le dispositif et agencé pour produire une résistance oscillatoire à la respiration à travers le dispositif, **caractérisé en ce que** l'appareil comprend une caméra électronique (20, 202) sensible au déplacement de l'élément déplaçable (10, 12), qu'au moins une partie de l'élément déplaçable (11, 12) est marquée d'une couleur contrastante ou d'une surface réfléchissante pour améliorer la sensibilité de la caméra (20, 202) au déplacement de l'élément déplaçable, que la caméra (20, 202) est montée à l'extérieur du dispositif (100), et que le dispositif comporte un boîtier transparent (2) ou une fenêtre transparente à travers laquelle la caméra (20, 202) peut visualiser l'élément déplaçable (11, 12) dans le dispositif.

2. Appareil de thérapie respiratoire selon la revendication 1, **caractérisé en ce que** la caméra (20, 202) est agencée pour fournir un signal indicatif d'un ou plusieurs des éléments suivants : lorsque le dispositif (100) est utilisé, la durée d'utilisation et la fréquence d'oscillation.

3. Appareil de thérapie respiratoire selon l'une quelconque des revendications précédentes, **caractérisé en ce que** la caméra (20, 202) est contenue dans une unité (200, 201) comportant un écran (205) sur lequel est représentée la fréquence d'oscillation détectée par la caméra.

4. Appareil de thérapie respiratoire selon la revendication 3, **caractérisé en ce que** la caméra est prévue dans un téléphone (200), et que le téléphone est programmé pour répondre au déplacement de l'élément déplaçable (11, 12) dans le dispositif (100) pendant l'utilisation.

5. Appareil de thérapie respiratoire selon l'une quelconque des revendications précédentes, **caractérisé en ce que** l'élément déplaçable comprend un élément de soupape (11) sur un culbuteur (12) qui ouvre et ferme une ouverture pendant l'expiration à travers le dispositif (100).

6. Appareil de thérapie respiratoire selon l'une quelconque des revendications précédentes, **caractérisé en ce que** l'appareil comprend également un microphone agencé pour détecter le son produit par le dispositif de thérapie, et **en ce que** l'appareil combine les signaux provenant de la caméra (20, 202) et du microphone pour améliorer le rapport signal/bruit.

7. Appareil de thérapie respiratoire selon l'une quelconque des revendications précédentes, **caractérisé en ce que** la caméra (20, 202) est agencée pour fournir une indication de la portée du dispositif de thérapie (100) à partir de la caméra, et **en ce que** l'appareil est agencé pour ajuster son gain en conséquence.
